# EUROPEAN PATENT APPLICATION

(11) **EP 0 789 032 A2**
(43) Date of publication of application: **13.08.1997**
(21) Application number: 97100856.0
(22) Date of filing: 21.01.1997
(51) Int. Cl.: C07K 16/40, C07K 16/38, G01N 33/573, G01N 33/53

(54) **Method for preparing a monoclonal antibody that provides an equimolar response to free and complexed analyte in a monoclonal/polyclonal sandwich immunoassay**

(30) Priority: 01.02.1996 US 595155
(71) Applicant: Bayer Corporation, Pittsburgh, PA 15219-2502 (US)
(72) Inventor: Zhou, Zeqi, New City, New York 10956 (US); Armstrong E. Glenn Jr., Stamford, Connecticut 06902 (US); Yeung, Kwok K., Ridgefield, Connecticut 06877 (US)
(74) Representative: Burkert, Frank

(57) **Abstract**

A method for preparing a monoclonal antibody which, when used in a monoclonal/polyclonal antibody sandwich immunoassay method for measuring an analyte which can exist in both free and binding protein complexed forms in a biological test sample, provides an equimolar response to the free and complexed forms of the analyte. The monoclonal antibody is selected to have the properties of (i) upon binding to the analyte, rendering the analyte substantially incapable of binding with antibodies that can bind the free form, but not the complexed form, of the analyte, and (ii) binding substantially equivalently with free and complexed analyte. The method is particularly useful in the measurement of prostate-specific antigen (PSA) in blood, serum or plasma.

## Description

### BACKGROUND OF THE INVENTION

The present invention relates to sandwich immunoassay methods for measuring an analyte which can east in both free and binding protein-complexed forms in a biological test sample such as blood, serum or plasma. More particularly, the invention relates to methods for measuring prostate-specific antigen (PSA) which provide an equimolar response to the free and complexed forms of PSA.

Human prostate-specific antigen (PSA) is a glycoprotein of approximately 33 kilodaltons (kDa) with a high amino acid sequence homology to the human kallikrein family (*1, 2*) and has been shown to be a serine protease with chymotrypsin and trypsin-like activity (*3-5*). PSA is secreted by the epithelial cells of the prostate gland and is one of the major proteins found in seminal fluid (*6*). Since the initial discovery of PSA in the serum of prostate cancer patients, there have been numerous reports regarding the role of this protein as an important, clinically useful biomarker in the management of patients with prostate cancer (*7-10*).
Recently, the American Cancer Society recommended that men aged 50 and older be screened annually for prostate cancer using serum PSA in conjunction with digital rectal examination (*11*).

Several laboratories have independently shown that PSA forms complexes with serine protease inhibitors such as I₁-antichymotrypsin (ACT) and I₂-macroglobulin (*12-19*). The majority of immunoreactive PSA found in human serum is complexed with ACT (*12*). Stenman *et al*. *(12)* and Lilja *et al*. (*17*) demonstrated that serum from prostate cancer patients contains a higher proportion of ACT-complexed PSA than that of benign prostate hyperplasia (BPH) patients. Recent studies have suggested that the quantification of free PSA, PSA-ACT complexes, or the determination of the ratio of free to total or PSA-ACT complex to total PSA in patient serum may differentiate prostate cancer from BPH (*12, 15, 16, 23*).

Epitope mapping studies with murine monoclonal antibodies have shown that there are five major, non-overlapping epitopes, named A through E, on the free PSA molecule (*20, 22*). One of these epitopes, Epitope E, has been observed to be completely masked when ACT binds to PSA. Thus, assays for free PSA can be developed by using antibodies directed against Epitope E. Another epitope, Epitope D, is considered to be partially blocked or to undergo a conformational change when ACT binds to PSA (*22*). This observation is based on the identification of a monoclonal antibody that binds to free PSA with seven times higher affinity than PSA-ACT complex and also prevents binding of other antibodies to Epitope E. In contrast, the other three epitopes, A, B and C have been observed to be essentially unaffected by the binding of ACT to PSA.

In the past several years, several laboratories have reported that commercial assays measuring serum PSA give different PSA values for the same patient specimens (*13, 19, 21*). For example, Graves *et al*. (*21*) found that the Yang Pros-Check® method yielded PSA values that were significantly higher than those from the Hybritech Tandem-R® assay on the same samples. Others have reported that, for some patient samples, the Ciba Corning ACS: 180^{ô} method and the Abbott IMx® method yielded PSA values approximately 2 to 3 times higher than the Hybritech Tandem-R PSA method (*13*). The reason for these differences his been attributed chiefly to variances in calibrator preparations and to the assay formats and antibodies used (*13, 20*).

Recently, Stamey reviewed the effect of immunoassay format on serum PSA quantification (*19*). He noted that assays using a monoclonal-monoclonal antibody sandwich format yielded, in general, equimolar quantifications of free and ACT-complexed PSA, whereas assays utilizing a monoclonal-polyclonal antibody format did not. The latter assays tended to yield higher values for the samples containing greater proportions of free PSA (*19*). McCormack *et al*. (*2*0) explained that the skewed response in non-equimolar response assays could be due to either the length of incubation time or the nature of the antibodies used in reagents. The suggestion was that for methods which use short incubation times, the antigen and antibodies do not achieve equilibrium kinetics, causing a greater response to free PSA than to PSA-ACT. Incubation time required for an equimolar response assay would thus vary depending on the assay format and antibody affinity constants for the two PSA forms.

Another factor which is understood to contribute to the non-equimolar response of monoclonal-polyclonal sandwich PSA assays relates to the characteristics of the polyclonal antibody. Polyclonal antibodies raised against free PSA contain a subpopulation of antibodies directed at a region (Epitope E) which is obscured when PSA is bound to ACT. The binding of ACT to PSA shields this epitope and prevents the binding of the sub-population of antibodies to PSA-ACT. Thus, fewer molecules of antibodies in the polyclonal antiserum bind to PSA-ACT, resulting in a lower response of the immunological method than is achieved when an equivalent molar concentration of free PSA is present, and thus a lower sample result.

Use of a non-equimolar response assay to detect PSA in patients suspected of having prostate cancer complicates interpretation of clinical results because a distinct cut-off of 4 ng PSA/ml serum is used to distinguish those patients with a higher risk of prostate cancer from those who are likely to be normal or have benign disease. In a non-equimolar method, results may be falsely above 4 ng/ml due to a higher percentage of free/total PSA in both normal patients and those with benign disease compared to patients with prostate cancer. This is not the case in an equimolar method in which a patient sample with 4 ng/ml or less would be consistently recovered as such regardless of the percentage of free/total PSA.

PCT Publication WO 95/18381 describes a monoclonal/polyclonal sandwich assay for an analyte such as PSA in which a third antibody is added in soluble form. Such third antibody has the property of being capable of binding with free analyte but not complexed analyte. As a result, it is reported that the response of the resulting immunoassay is rendered equimolar. There is no suggestion of an immunoassay method in which selection of the binding properties of the monoclonal antibody involved in sandwich formation results in an equimolar response and without the addition of a further reagent.

### SUMMARY OF THE INVENTION

It has now been unexpectedly found that judicious selection of the monoclonal antibody to be used in a monoclonal/polyclonal sandwich immunoassay for an analyte that exists in both free and binding protein complexed forms in a biological test sample provides an assay response that is equimolar for free and complexed analyte. The selected monoclonal antibody should, upon binding to the analyte, substantially block the binding of antibodies that can bind the free form, but not the complexed form, of the analyte, and further should bind substantially equivalently to free and complexed analyte. Such binding properties assure that the polyclonal antibody will bind to free analyte to the same extent as complexed analyte since binding of the monoclonal antibody to free analyte effectively blocks the same epitopes as are blocked by complexation with the binding protein.

In one aspect, the present invention provides a method for preparing such monoclonal antibody comprising the steps of (a) preparing a population of hybridomas by somatic cell fusion of myeloma cells with lymphocyte cells from an animal that has been appropriately immunized against the analyte, (b) screening the hybridoma population to isolate a hybridoma cell line that produces a monocloral antibody which (i) upon binding to the analyte, substantially blocks the binding of antibodies that can bind the free form, but not the complexed form, of the analyte, and (ii) binds substantially equivalently to free and complexed analyte, and (c) producing the monoclonal antibody from said isolated hybridoma.

The monoclonal antibody of the present invention is particularly useful in mixed sandwich immunoassays wherein a liquid test mixture is formed by combining the test sample with an anti-analyte monoclonal antibody and an anti-analyte polyclonal antibody, one of such antibodies being labeled ("labeled antibody") and the other being immobilized or capable of being immobilized for purposes of separation from the liquid test mixture ("capture antibody"), the capture antibody is separated from the liquid test mixture, and the label is measured in one of the resulting separated phases.

The analyte and its corresponding binding protein are preferably selected from prostate-specific antigen (PSA) and I₁-antichymotrypsin (ACT); PSA and I₁-protease inhibitor; PSA and protein C inhibitor; protein C and protein C inhibitor; elastase and anti-trypsin; cathepsin G and ACT; thrombin and anti-thrombin III; C₁-esterase and C₁-inhibitor; t-PA and PAI-1; uPA and PAI-1; and plasmin and I₂-antiplasmin. The present invention is particularly useful for the measurement of prostate-specific antigen (PSA), particularly when the binding protein of interest is I₁-antichymotrypsin (ACT).

### BRIEF DESCRIPTION OF THE DRAWINGS

Figure 1 is a graph showing the specificity of representative monoclonal antibodies (PSA30 and PSA66).

Figure 2 is a graph showing inhibition of binding of PSA30 to free PSA by various antibodies.

Figure 3 is a graph demonstrating that monoclonal antibody MM1 inhibits binding of PSA30 to an epitope (Epitope E) on PSA that is masked in the PSA-ACT complex.

Figure 4 is a graph showing that the combination of MM1-FITC and MP2-ALP produces an equimolar response immunoassay for free and ACT-complexed PSA.

### DESCRIPTION OF THE PREFERRED EMBODIMENTS

The monoclonal antibody of the present invention can be prepared in a number of ways. Principally, the monoclonal antibody will be prepared by applying conventional somatic cell hybridization techniques using a screening method for selection of hybridoma cell lines which results in isolation of hybridomas which produce a monoclonal antibody having the defined binding properties of the present invention. The strategy for such screening is to select antibodies that block the binding of other antibodies directed to epitopes accessible on free PSA but not complexed PSA, e.g., Epitope E, but themselves have substantially equivalent binding to free analyte and binding protein complexed analyte.

Somatic cell hybridization is now a well-known methodology and can be applied to the present invention in all of its variations as appropriate and desired. In general, one prepares a population of hybridomas by fusion of myeloma cells with lymphocyte cells taken from an animal that has been immunized against the analyte. Immunization of the host animal, as used herein, implies that the animal's immune system has been challenged to produce antibodies that will bind to one or more epitopes on the analyte of interest. It will be evident to the skilled worker in the art that such result can be obtained in any number of ways, including, without limitation, administration of the native analyte, synthetic peptide immunogen, tranfectant cells which express epitopes of the analyte on their surface, or the like, to the bloodstream of the host animal. Similarly, production and harvesting of monoclonal antibodies from cloned hybridoma cell lines are within the ordinary skill in the art, and in general any known method can be used in practicing the present invention.

As described above, the principal criteria for screening of hybridomas to produce the monoclonal antibody of the present invention are: (i) that it produce a monoclonal antibody which, upon becoming bound to the analyte, renders the analyte substantially incapable of binding to antibodies that are specific for the free form of the analyte relative to the complexed form of the analyte, that is, antibodies that can bind the free form, but not the complexed form, of the analyte, and (ii) that such monoclonal antibody have the property of substantially equivalent binding to free analyte and complexed analyte. In this way, the free and complexed forms of the analyte, when bound by the monoclonal antibody of the present invention, are indistinguishable with respect to their ability to be bound by the antibodies in the polyclonal population which are directed to epitopes available only in the free form of the analyte, i.e., epitopes which are masked or obscured or altered when the analyte is complexed to the binding protein of interest. The mechanism by which the monoclonal antibody of the present invention operates to provide the above result is not clearly understood, however, it is speculated that the binding of the monoclonal antibody to the free form of the analyte itself blocks, masks, obscures, or alters the epitope(s) that are available only on free analyte (but not complexed analyte) for binding by an antibody or antibodies directed to such epitope(s).

The monoclonal antibody of the present invention imparts an equimolar response to monoclonal/polyclonal ("mixed") sandwich immunoassays for measuring analytes that exist in free and complexed forms in the test sample. In general, it will be understood that such sandwich assays involve the basic steps of combining the test sample with two different antibody reagents generally referred to herein as the labeled antibody and the capture antibody.

The labeled antibody (or sometimes referred to as the detection antibody) is anti-analyte antibody incorporated with a chemical moiety or label which is directly or indirectly detectable. Commonly, such label moiety possesses a detectable physical property, e.g., radioactivity, fluorescence, or the like, or is detectable by chemical reaction or binding, e.g., an enzyme, chemiluminescent reactant, or ligand which can be bound by a binding partner or receptor which itself is labeled for detection (such ligand/receptor pairs being exemplified by biotin/avidin and hapten/anti-hapten antibody). Such labels and detection means are now well known in the art.

The capture antibody similarly can vary in accordance with the known art. The understood essential property of the capture antibody is that it provide a means for separation from the liquid reaction mixture in order to provide separation of unbound labeled antibody from sandwich complexes that are formed by binding of the capture and labeled antibodies to the analyte of interest. Typically, this is accomplished by employing an immobilized or insolubilized form of the capture antibody, e.g., antibody linked to a solid surface, macro or microparticle, or other solid phase, or by employing antibody modified with a moiety that provides for rendering the antibody immobilized or insolubilized subsequently to the immunoassay reaction, for example, by incorporating a ligand which can be bound by an immobilized binding partner or receptor (such ligand/receptor pairs being exemplified, as above, by biotin/avidin and hapten/anti-hapten antibody). Such means for attaining separation of the free and bound forms of the labeled antibody are also now well known in the art.

In the immunoassay method, after combining and appropriately incubating the test sample with the labeled and capture antibodies, the capture antibody (and complexes of the analyte with labeled antibody which have become bound to the capture antibody) are separated from the remaining liquid mixture. Thereafter, the label is measured in an appropriate manner in one of the separated phases, usually the capture antibody phase. In general, the anti-analyte monoclonal antibody can be used as either the labeled antibody or the capture antibody so long as conditions are selected to optimize the equimolar response provided by the present invention. However, it will generally be preferred to combine the monoclonal antibody reagent with the test sample before, or simultaneously with the polyclonal antibody reagent.

The present invention also provides a test kit for use in performing the immunoassay method of the present invention. Such test kit will comprise a packaged combination or configured device comprising the labeled and capture antibodies wherein one is the monoclonal antibody of the present invention and the other is a polyclonal antibody as defined herein. Examples, without limitation, of such test kits are a packaged combination of one or more containers (e.g., tubes, chambers, or the like) holding the labeled and capture antibodies; and test devices comprising the labeled and capture antibodies in a liquid or solid, dry state. A great many test kit formats are known in the art and any convenient or advantageous format can be selected by the skilled worker according to need.

The present invention is applicable to immunoassays for the determination of essentially any analyte of interest which exists in a biological test sample in free and complexed forms. Primarily, the free form of the analyte will be understood to mean the analyte unbound to a particular binding or carrier protein in the test sample, whereas the complexed form means the analyte in the condition of being bound or complexed with such particular binding protein. However, it will be further understood that the present invention is applicable in general to any assay based 0:0 specific binding reagents in which the analyte of interest can be in a free form as well as bound or complexed with a counterpart binding partner. In regard to analyte/binding protein pairs of principal interest to the practice of the present invention, the following can be specifically mentioned: (PSA) and I₁-antichymotrypsin (ACT); PSA and I₁-protease inhibitor; PSA and protein C inhibitor; protein C and protein C inhibitor; elastase and anti-trypsin; cathepsin G and ACT; thrombin and anti-thrombin III; C₁-esterase and C₁-inhibitor; t-PA and PAI-1; uPA and PAI-1; and plasmin and I₂-antiplasmin. Especially useful is the application of the present invention to the measurement of PSA since the present invention provides means for obtaining an equimolar response to PSA and ACT-PSA complex using a monoclonal/polyclonal sandwich immunoassay.

The present invention will now be illustrated, but is not intended to be limited by, the following examples.

### EXAMPLES

### Materials

***Antibodies*** - Antibodies used included the following:
(1) PSA19 (Lot #5491), PSA30 (Lot #4752) and PSA66 (Lot #4403) monoclonal antibodies were purchased from CanAg Diagnostics AB (Gothenburg, Sweden). They were generated from mouse hybridoma clones by *in vitro* cultivation, and purified by affinity chromatography. Their purity was tested by SDS-PAGE and the visible bands were seen corresponding to their heavy and light chains under reducing conditions using the Pharmacia Fast System. The antibodies were stored in a buffer containing 0.9% sodium chloride. PSA19 and PSA30 specifically bind Epitope E of free PSA and PSA66 binds both free and I₁-antichymotrypsin (ACT)-complexed PSA.
(2) MM1, monoclonal antibody (Lot #CMO041594) and MP2, polyclonal antibody (Lot #JK112894) are the capture antibody and detection antibodies, respectively, used in the Technicon Immuno 1® PSA assay sold commercially in Europe by Bayer Corporation, Tarrytown, NY, USA). Such assay provides an equimolar response with free and ACT-complexed PSA.
(3) The antibodies used as controls include rabbit anti-recombinant glutathione S-transferase (GST, expressed in *E. coli*) polyclonal antibody which was purified from rabbit sera by Protein-A chromatography (Batch #T1L2) and purchased from Amard (Kew Victoria, Australia). Rabbit anti-human plasma ACT polyclonal antibody (Lot #8E1444) was from BioDesign (Kennebunk, Maine, USA).

***Antigens*** - Antigens used in these studies include the following: Free prostate-specific antigen (Lot #751464) and PSA-ACT (Lot #145564) were purchased from Scripps (San Diego, California, USA). Free PSA was purified from human seminal fluid with 98% purity by sodium dodecyl sulfate-gel electrophoresis (SDS-PAGE) and stored in a buffer containing 10 mM Tris, 0.1% sodium azide, pH 8.0. PSA-ACT was purified from human seminal fluid and plasma with >96% purity by SDS-PAGE and stored in a buffer containing 10 mM sodium acetate, 150 mM sodium chloride, and 0.1% sodium azide, pH 5.6.

### Buffer and Solution

(1) GS-5 buffer (Bayer Corporation Product No: T65-1954-01) containing 100 mM Tris-HCl was used for reagent dilution. This buffer contains 5% (heat-inactivated) normal goat serum (Lot #4001234) which was purchased from Biocell Laboratories (Carson, California, USA).
(2) 6% bovine serum albumin (BSA) solution with 25 mM Tris, 111 mM sodium chloride and 0.1% NaN3, pH 8.0 (Bayer Corporation Product No: T99-7040-01; Lot No: 94B03067) was used as a PSA calibrator base and for antigen dilutions. BSA was purchased from Sigma (St. Louis, Missouri, USA).

### Methods

***The Technicon Immuno 1***®***PSA Assay*** - The Technicon Immuno 1 PSA assay is a sandwich assay which uses a monoclonal antibody for capture and a polyclonal antibody for detection of PSA. The monoclonal anti-PSA antibody is conjugated to fluorescein (R1, 1.5 Tg/mL) and the affinity-purified polyclonal antibody is conjugated to alkaline phosphatase (ALP) (R2, 6.5 Tg/mL). Solutions of the two antibodies, at 65 Tl/test, are incubated with a test sample and the immunocomplex (R1-PSA-R2) is captured by magnetic particles (10 Tl/test) coated with anti-fluorescein monocloral antibodies. The immunocomplex is washed and then 300 Tl/test of 23 mM *p*-nitrophenyl phosphate substrate is added. Calibration of the Technicon Immuno 1® Analyzer (Bayer Corporation) is performed using the Technicon Immuno 1 SETpoint® PSA calibrators, prepared from free PSA at concentrations of 0, 2, 10, 25, 50 and 100 ng/mL. A cubic-through-zero fitting algorithm is used to generate a standard calibration curve. Operation of the Technicon Immuno 1 Analyzer is described in the manufacturer's instructions (24).

***The Technicon Immuno 1***® ***Free PSA Assay*** - The protocol used for the Technicon Immuno 1® free PSA assay was the same as that described above for the Technicon Immuno 1® total PSA assay except for the following changes: Monoclonal antibody (PSA30) specific for free PSA was conjugated to fluorescein (PSA30-FITC) to replace MM1-FITC for capture (R1), and combined with MP2-ALP to form a monoclonal-polyclonal antibody sandwich format on the Technicon Immuno 1 Analyzer. ACT-complexed PSA was obtained from Scripps (*supra*). The concentrations of R1 (65 Tl/test) and R2 (65 Tl/test) were 10 Tg /mL and 6.5 Tg/mL, respectively. The sample volume was 55 Tl/test and the magnetic particle volume was 20 Tl/test.

***Specificity Studies of PSA30 and PSA66 Monoclonal Antibodies*** - The assay was conducted on the Technicon Immuno 1 Analyzer as described for the Technicon Immuno 1 PSA assay except that PSA30-FITC (10 Tg/mL) and PSA66-FITC (1.5 Tg/mL) were used as R1. Free PSA or PSA-ACT complex at the concentrations of 0, 2, 4, 10 and 20 ng/mL were spiked into two SETpoint calibrators; Cal 1 and Cal 3 contain 0 and 10 ng/mL of free PSA, respectively. These solutions were pipetted as samples and the PSA concentration measured.

***Inhibition of Binding of PSA30-ALP to PSA by Various Antibodies*** - This assay was conducted on the Technicon Immuno 1 Analyzer as described above for the Technicon Immuno 1 PSA assay. PSA-FITC (0.5 Tg/mL, 65 Tl/test) was used as R1 and PSA30-ALP (0.2 Tg/mL, 65 Tl/test) as R2. Various concentrations from 0 to 40 Tg/mL of unlabeled antibodies were added as samples. The following antibodies were used for inhibition of binding of PSA30-ALP to PSA-FITC: PSA30, serials 1; PSA66, serials 2; MP2, serials 3; MM1, serials 4; and rabbit anti-GST, serials 5.

***Studies of MM1 Inhibition of PSA30 Binding to Epitope E*** - The assay was carried out on the Technicon Immuno 1 Analyzer as described above for the Technicon Immuno 1 PSA assay except that MM1-FITC as R1 was used at 1 Tg/mL (65 Tl/test) and PSA30-ALP (4 Tg/mL, 65 Tl/test) as R2. SETpoint free PSA calibrators (0-100 ng/mL, 20 Tl/test) were used as samples.

***Studies of Equimolar Response Assays for Free and ACT-Complexed*** ***PSA Using Different Antibody Combinations*** - The assay was conducted on the Technicon Immuno 1 Analyzer as described in Methods. In two of the methods, MM1-FITC was replaced with PSA66-FITC (1.5 Tg/mL) and PSA30 (10 Tg/mL). Free PSA and PSA-ACT complex were made up in various proportions (100%:0%, 80%:20%, 50%:50%, 20%:80% and 0%:100%) to give a final concentration of approximately 4 ng/mL. These PSA solutions (20 Tl/test) were analyzed as samples.

### Results

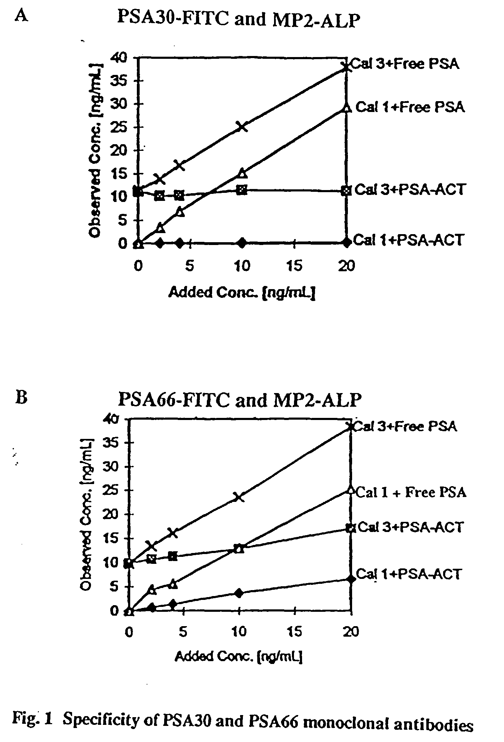

***Specificity of PSA30 and PSA66 Monoclonal Antibodies*** **-** Figures 1A and 1B show that both sandwich immunoassays (PSA30-FITC as R1 and MP2-ALP as R2; and PSA66-FITC as R1 and MP2-ALP as R2) gave mostly a linear response with increased levels of free PSA. This phenomenon was true for both levels of calibrators. The combination of PSA30-FITC as R1 and MP2-ALP as R2, however, showed no increased response with increased PSA-ACT concentration. These data indicate that PSA30 binds to Epitope-E and is specific for free PSA. In contrast, the PSA66-FITC and MP2-ALP combination gave increased response with increased PSA-ACT levels. These data show that PSA66 binds an epitope on PSA other than Epitope E. Thus, PSA66 binds to both free PSA as well as PSA-ACT complex.

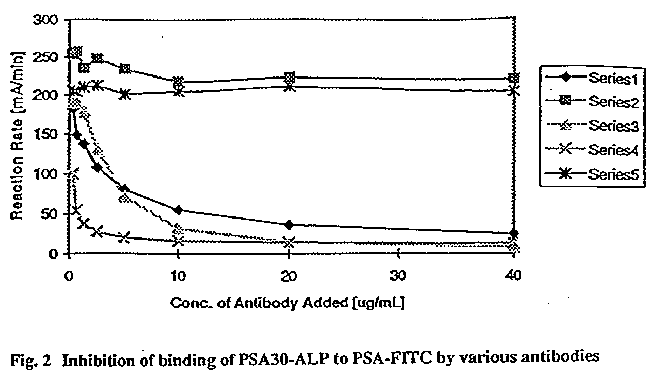

***Inhibition of Binding of PSA30-ALP to PSA by Various*** ***Antibodies*** - PSA30, MP2 and MM1 all competed effectively for the binding of PSA30-ALP to PSA as shown in Fig. 2. Surprisingly, the slope of the binding inhibition curve using MM1 is even steeper than that for PSA30, suggesting that MM1 blocks PSA30-ALP binding even more effectively than PSA30 itself. PSA66 which binds to PSA but is not specific for Epitope E showed insignificant inhibition. An irrelevant polyclonal antibody, rabbit anti-GST, did not inhibit the binding of PSA30-ALP to PSA-FITC. These data show that MP2 contains antibodies which bind to Epitope E.

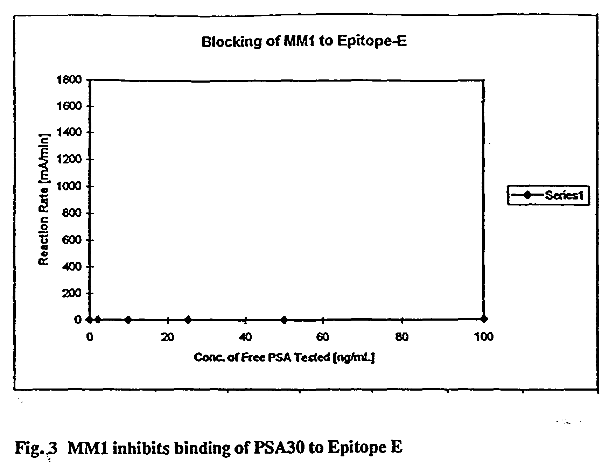

***Blocking of Epitope E binding of PSA30 by MM1*** - Results show that no immunoreactivity was detected at the several levels of PSA added (Fig. 3). This suggests that no immunocomplex was formed with MM1, PSA and PSA30. One explanation for this is that the epitopes which MM1 and PSA30 bind are so close together that the binding of one antibody to PSA sterically inhibits the binding of the other. Another explanation is that the binding of MM1 changes the conformation of Epitope E on PSA so that PSA30 no longer recognizes it.

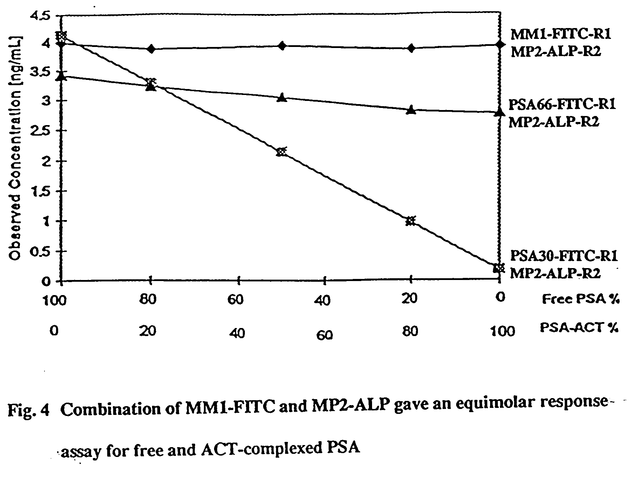

***Combination of MM1-FITC and MP2-ALP Provided An Equimolar Response Assay for Free and ACT-complexed PSA*** - MM1-FITC in the Technicon Immuno 1 PSA assay was replaced with either PSA66-FITC or PSA30-FITC for capture. All these methods were assayed with mixtures with various proportions of free and ACT-complexed PSA. The Fig. 4 results show that the Technicon Immuno 1 PSA assay which uses the monoclonal antibody, MM1-FITC, in combination with the polyclonal antibody, MP2-ALP, provides an equimolar response for free and ACT-complexed PSA. Based on the observations reported here, this is most likely due to MM1 blocking a subpopulation of antibodies which are directed to Epitope E in the polyclonal antiserum, from binding to Epitope E in the free PSA molecule. Another possibility is that when MM1 binds to PSA, the protein undergoes conformational changes such that Epitope E specific antibodies in MP2 no longer bind to this epitope. In contrast, PSA66 which does not inhibit the binding of this antibody subpopulation to Epitope E, gives a skewed response, reflecting greater reactivity of free PSA than PSA-complex in the method. Fig. 4 also shows that the combination of PSA30-FITC which binds to Epitope-E as R1 and MP2-ALP as R2 provides a specific test for free PSA.

The Technicon Immuno 1 PSA method which uses a monoclonal capture antibody and a polyclonal detection antiserum has thus been demonstrated to unexpectedly provide an equimolar response. The above studies have shown that this unexpected finding is not due to the binding properties of the polyclonal antiserum (MP2) because when it is paired with monoclonal antibodies other than MM1, it binds preferentially free PSA thereby causing a skewed response. This is likely due to the presence of antibodies in MP2 to Epitope E on PSA. In actuality, the equimolar response is due to the properties of the monoclonal antibody (MM1) which excludes the binding of antibodies to Epitope E and still binds equally well to free PSA and PSA-ACT complex. Monoclonal antibodies with this unique combination of properties previously have not been described. Antibodies which bind to Epitope D have been found to inhibit the binding of those to Epitope E, but also bind with greater affinity to free PSA than PSA-ACT complex (*22*).

The present invention has been particularly described and exemplified above. Clearly, many other variations and modifications of the invention can be made without departing from the spirit and scope hereof.

### BIBLIOGRAPHY

1. Wang MC, Valenzuela LA, Murphy GP, Chu TM. Purification of a human prostate specific antigen. Invest Urol 1979; 17:159-63.
2. Watt WK, Lee PJ, Timkulu TM, Chan WP, Loor R. Human prostate-specific antigen: structure and functional similarity with serine proteases. Proc Natl Acad Sci USA 1986; 83:3166-70.
3. Akiyama K, Nakamura T, Iwanaga S, Hara M. The chymotrypsin-like activity of human prostate-specific antigen, r-seminoprotein. FEBS Letters 1987; 225:168-72.
4. Christensson A, Laurel CB, Lilja H. Enzymatic activity of prostatic-specific antigen and its reactions with extracellular serine proteinase inhibitors. Eur J Biochem 1990; 194:755-763.
5. Lilja H. A kallikrein-like serine protease in prostatic fluid cleaves the predominant seminal vesicle protein. J Clin Invest 1985; 76:1899-903.
6. Lilja H, Abrahamsson PA. Three predominant proteins secreted by the human prostate gland. Prostate 1980; 12:29-38.
7. Papsidero LD, Wang MC, Valenzuela lA, Murphy GP, Chu TM. A prostate antigen in sera of prostate cancer patients. Cancer Res 1980; 40:2428-2432.
8. Lange PH. Prostate specific antigen in diagnosis and management of prostate cancer. Supplement to Urology 1990; XXXVI:25-29.
9. Oesterling JE. Prostate specific antigen: a critical assessment of the most useful tumor marker for adenocarcinoma of the prostate. J Urol 1991; 145:907-923.
10. Armbruster DA. Prostate-specific antigen: biochemistry, analytical methods and clinical application. Clin Chem 1993; 39:181-195.
11. American Cancer Society: Cancer facts & figures-1995. American Cancer Society, Inc., Atlanta, GA. Page 11.
12. Stenman UH, Leinonen J, Alfthan H, Rannikko S, Tuhkanen K, Alfthan O. A complex between prostate-specific antigen and I121-antichymotrypsin is the major form of prostate-specific antigen in serum of patients with prostatic cancer: assay of the complex improves clinical sensitivity for cancer. Cancer Res 1991; 51:222-226.
13. Zhou AM, Tewari PC, Bluestein BL, Caldwell, GW, Larsen FL. Multiple forms of prostate-specific antigen in serum: differences in immunorecognition by monoclonal and polyclonal assays. Clin Chem 1993; 39:2483-91.
14. Leinonen J, Lovgren T, Vornanen T, Stenman UH. Double-label time-resolved immunofluorometric of prostate-specific antigen and its complex with I₁-antichymotrypsin. Clin Chem 1993: 39:2098-2103.
15. Christensson A, Bjork T, Nilsson O, Nilsson O, Dahlen U, Matikainen MT, Cockett AT, Abrahamssion PA. Serum prostate specific antigen complexed with I₁-antichymotrypsin as an indicator of prostate cancer. J Urol 1993; 150:100-105.
16 Lilja H, Significance of different molecular forms of serum PSA. The free, noncomplexed forms of PSA versus that complexed to I₁-antichymotrypsin. Urol Clin North Am 1993; 20(4):681-686.
17. Lilja H, Christensson A. Dahlen U, Matikainen M-T, Nilsson O, Pettersson K and Lovgren T: Prostate-specific antigen in human serum occurs predominantly in complex with alpha-antichymotrypsin. Clin Chem 1991; 37:1618-1625.
18. Bjork T, Hulkko S, Bjartell A, Santagnese AD, Abrahamsson P-A, Lilja H. Alpha₁-antichymotrypsin production in PSA-producing cells is common in prostate cancer but rare in benign prostatic hyperplasia. Urology 1994; 43:427-434.
19. Stamey TA. Second Stanford conferences on international standardization of prostate specific antigen immunoassays: September 1 and 2, 1994. Urology 1995; 45:173-184.
20. McCormack RT, Rittenhouse HG, Finlay JA, Sokoloff RL, Wang TJ, Wolfert RL, Lilja H, Oesterling JE. Molecular forms of prostate-Specific antigen and the human kallikrein gene family: a new era. Urol 1995; 45:729-744.
21. Graves HCB, Wehener N, Stamey TA. Comparison of a polyclonal and monoclonal immunoassay for PSA: need for an international antigen standard. J Urol 1990; 144:1516-1522.
22. Pettersson K, Piironen T, Seppala M, Liukkonen L, Christensson A, Matikainen M-T, Suonpaa M, Lovgren T, Lilja H. Free and complexed prostate-specific antigen (PSA): in *vitro* stability, epitope map, and development of immunofluorometric assays for specific and sensitive detection of free PSA and PSA-I₁-antichymotrypsin complex. Clin Chem 1995; 41:1480-1488.
23. Wu J T. Assays for prostate specific antigen (PSA): problems and possible solutions. J Clin Lab Anal 1994; 8:51-62.
24. Technicon Immuno 1® Assay Instructions. Miles Inc., Clinical Method (DA4-1207F94), June 1994.

## Claims

1. A method for preparing a monoclonal antibody to an analyte which can exist in both free and binding protein complexed forms in a biological test sample, the monoclonal antibody, when used in a monoclonal/polyclonal sandwich immunoassay method, provides an equimolar response to the free and binding protein complexed forms of the analyte present in said test sample, comprising the steps of:
(a) preparing a population of hybridomas by somatic cell fusion of myeloma cells with lymphocyte cells from an animal that has been immunized against said analyte,
(b) screening said hybridoma population to isolate a hybridoma cell line that produces a monoclonal antibody which (i) upon binding to the analyte, renders the analyte substantially incapable of binding to antibodies that can bind the free form, but not the complexed form, of the analyte, and (ii) binds substantially equivalently to free and complexed analyte, and
(c) producing said monoclonal antibody from said isolated hybridoma.

2. The method of claim 1 wherein the analyte and its corresponding binding protein are selected from prostate-specific antigen (PSA) and I₁-antichymotrypsin (ACT); PSA and I₁-protease inhibitor; PSA and protein C inhibitor; protein C and protein C inhibitor; elastase and anti-trypsin; cathepsin G and ACT; thrombin and anti-thrombin III; C₁-esterase and C₁-inhibitor; t-PA and PAI-1; uPA and PAI-1; and plasmin and I₂-antiplasmin.

3. The method of claim 1 wherein the analyte is prostate-specific antigen (PSA) and the binding protein is I₁-antichymotrypsin (ACT).

4. The method of claim 1 wherein the biological test sample is blood, serum or plasma.

5. A monoclonal antibody prepared according to the method of any one of claims 1-3.

6. In a method for measuring an analyte in a biological test sample, which analyte can exist in both free and binding protein complexed forms in said test sample, wherein a liquid test mixture is formed by combining said test sample with an anti-analyte monoclonal antibody and an anti-analyte polyclonal antibody, one of said antibodies being labeled ("labeled antibody") and the other being immobilized or capable of being immobilized for purposes of separation from the liquid test mixture ("capture antibody"), said capture antibody is separated from the liquid test mixture, and the label is measured in one of the resulting separated phases,
the improvement which comprises employing as said monoclonal antibody, the monoclonal antibody of claim 5, whereby the assay method provides an equimolar response to the free and binding protein complexed forms of the analyte present in said biological test sample.

7. The method of claim 6 wherein the monoclonal anti-analyte antibody is combined with the test sample before, or substantially simultaneously with, the polyclonal anti-analyte antibody.

8. A test kit for use in a monoclonal/polyclonal immunoassay method for measuring an analyte which can exist in both free and binding protein complexed forms in a biological test sample, comprising an anti-analyte monoclonal antibody and an anti-analyte polyclonal antibody, one of said antibodies being labeled ("labeled antibody") and the other being immobilized or capable of being immobilized for purposes of separation from the liquid test mixture ("capture antibody") formed upon combination of said test sample and said labeled and capture antibodies, wherein said monoclonal antibody is the monoclonal antibody of claim 5.
